# EUROPEAN PATENT APPLICATION

(11) **EP 4 420 662 A1**
(43) Date of publication of application: **28.08.2024**
(21) Application number: 22882737.4
(22) Date of filing: 13.10.2022
(51) Int. Cl.: A61K 31/433, A61K 31/497, A61P 13/12, A61K 31/438

(54) **USE OF TACHYKININ RECEPTOR 3 INHIBITOR**

(30) Priority: 18.10.2021 CN 202111210124
(71) Applicant: Wuhan University, Wuhan, Hubei 430072 (CN)
(72) Inventor: SONG, Wei, Wuhan, Hubei 430072 (CN); XU, Wenhao, Wuhan, Hubei 430072 (CN); LI, Gerui, Wuhan, Hubei 430072 (CN)
(74) Representative: Plougmann Vingtoft a/s
(86) International application number: PCT/CN2022/125106
(87) International publication number: WO 2023/066127

(57) **Abstract**

The present invention provides a use of an NK3R inhibitor in preparation of a drug, the drug is used for treating chronic kidney diseases, and the structure of the NK3R inhibitor is as shown in Formula (I) or Formula (II).

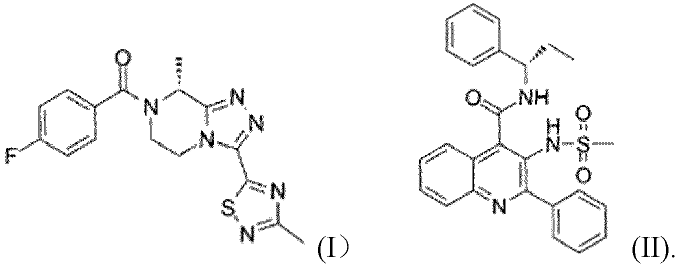

## Description

### Field of the Invention

The present invention relates to novel uses of compounds, in particular to novel uses of tachykinin receptor 3 (NK3R) inhibitors.

### Background of the Invention

### 1.1 Chronic Kidney Disease (CKD)

Chronic kidney disease (CKD) has become a worldwide public health problem. The prevalence of adult chronic kidney disease in China was as high as 11% in 2016, and the growth rate of chronic kidney disease will exceed 17% in the next decade. Chronic kidney diseases refer to chronic kidney structural and functional disorders (history of kidney damage more than 3 months) caused by various reasons, including renal tubular lesions, pathological damage leading to abnormal glomerular filtration rate (GFR), abnormal blood or urine composition, abnormal imaging results, or unexplained decrease in GFR (<60ml/min·1.73m²) for more than 3 months. Chronic kidney disease is irreversible, and most kidney disease patients need to rely on and receive long-term medication to control their development. In 2016, the market size of China's kidney disease drugs was 28.74 billion yuan, and the market size of global drugs was 151.98 billion dollars. The major risk factors leading to chronic kidney diseases are hypertension, diabetes, cancer, aging and pharmacologic damage, and so on.

### 1.2 Cancer and Kidney Disease

The number of cancer patients increases to nearly 9 million in the world as the result of new cases and prolonged patients' survival time, 60% of which are malignant. For nearly one quarter of cancer patients ^{[1][4]}, the direct cause of death is tumor-induced complications, including cancer-related kidney damage. Cancer-related kidney damage includes chronic kidney disease and acute kidney damage, which may arise from direct damage to the kidneys by chemotherapeutic agents, invasion and compression of the urinary system by tumors, and pre-renal kidney damage caused by systemic depletion ^{[5]}. On the other hand, end-stage kidney disease (uremia) leads to metabolic and immune disorders in body, which will also cause and aggravate organ failure, and the combined chronic kidney disease will also reduce the survival rate of cancer patients. Therefore, the comprehensive treatment and management of cancer patients with combined kidney damage also need to be reasonably optimized, and the protection of kidney function and the treatment of kidney damage also need to be paid attention to. However, because the pathogenesis of kidney damage caused by cancer is unknown, its treatment has been mainly palliative ^{[2]}, and even in the United States, the Food and Drug Administration has not approved any drug for the treatment of cancer complications^{[3]}. Therefore, drugs for the treatment of cancer-related kidney disease need further research and development.

### 2. NK3R inhibitors

### 2.1 Function and expression of NK3R

NK3R (tachykinin receptor 3) is a G-protein-coupled receptor, a molecule that has been widely studied in the fields of growth and reproduction. NK3R is expressed in various mammalian tissues. Among them, NK3R expressed in the nervous system has been shown relating to a variety of neurological diseases, such as mood disorders, Alzheimer's disease, Parkinson's disease, and schizophrenia^{[6]}. NK3R is also highly expressed in tissues such as kidney and bladder, but its impact on the urinary system is unknown. For the first time, we discovered that NK3R is an important regulatory factor that mediates tumor-induced damage to kidney function, and inhibition of NK3R greatly improved kidney function without affecting tumor growth.

### 2.2 Information on small molecules inhibiting NK3R

Fezolinetant is an effective NK3R selective inhibitor with a molecular formula of C₁₆H₁₅FN₆OS, a molecular weight of 358.4 g/mol, and a structural formula shown in Formula (I). The Ki of NK3R inhibition in vitro is 25 nM, and the IC50 is 20 nM. Phase 2 clinical trials found that it has a significant improvement effect on hot flashes in postmenopausal women^{[8]}, and phase 2 clinical trials for polycystic ovary syndrome are ongoing.

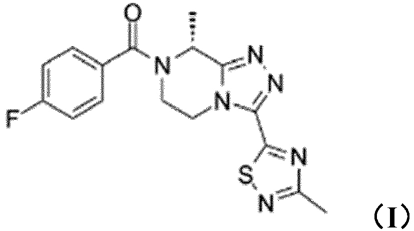

Pavinetant is another effective NK3R selective inhibitor with a molecular formula of C₂₆H₂₅N₃O₃S, a molecular weight of 459.6 g/mol and a structural formula shown in Formula (II). The in vitro inhibitory IC50 was found to be 7.1 and 19.8 µM by midazolam and testosterone testing in cytochrome P450-3A4 enzyme activity assay, respectively. Phase 2 clinical trials found that it significantly improves polycystic ovary syndrome and hot flashes^{[7]}.

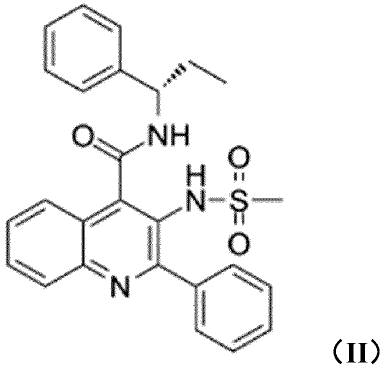

However the effect of blocking NK3R with these two inhibitors on kidney function has not yet been reported.

### Summary of the Invention

It is an object of the present invention to provide novel uses of NK3R inhibitors.

Previous studies have found that the application of these two inhibitors in tumor-bearing Drosophila and mouse models can promote urination, improve kidney function, reduce kidney damage and ascites generation. The results indicate that the NK3R inhibitors Pavinetant and Fezolinetant may be potentially effective drugs for the treatment of tumor-induced kidney damage.

According to one aspect of the present invention, there is provided the use of an NK3R inhibitor in the preparation of a drug. The drug is used for the treatment of chronic kidney diseases, and the NK3R inhibitor has the structure shown in formula (I) or formula (II).

In a preferred embodiment of the present invention, the NK3R inhibitor is used for the treatment of chronic kidney diseases. The chronic kidney disease is selected from the group consisting of glomerulonephritis, latent nephritis, pyelonephritis, allergic purpura nephritis, lupus erythematosus nephritis, gouty nephropathy, nephrotic syndrome, membranous nephropathy, nephrotic syndrome, diabetic nephropathy, hypertensive nephropathy, and polycystic kidney.

According to another aspect of the present invention, the present invention provides the use of an NK3R inhibitor in the preparation of a drug. The drug is used for the treatment or alleviation of tumor-induced renal tubular damage, and the NK3R inhibitor has the structure shown in Formula (I) or Formula (II).

More preferably, the NK3R inhibitor of the present invention is used for the treatment or prevention of kidney diseases caused by advanced tumors.

The present invention finds that two NK3R inhibitors, Fezolinetant and Pavinetant, can be used to reduce tumor-induced renal tubular damage.

Experiments on Drosophila demonstrated that overexpression of the activated proto-oncogene yki in Drosophila intestinal stem cells can induce significant hyperglycemia and ascites accumulation. Feeding with food supplemented with the NK3R inhibitors Fezolinetant (100 µM) and Pavinetant (100 µM) for 6 days can significantly reduce ascites in Drosophila, and normalize blood glucose. C57BL/6J-APCMin/+ mice are well-established intestinal adenoma model. Further experiments revealed that NK3R was highly expressed in kidney tubules. More importantly, C57BL/6J-APCMin/+ mice began to show weight loss at 16 weeks of age and showed decrease of urine output by about 30% compared to that of normal C57BL/6J mice, and kidney HE staining revealed severe damage of the renal tubules. Intraperitoneal injection of the NK3R inhibitor Fezolinetant (10 mg/kg/day ) for 9 days significantly restored urinary output and renal tubular damage including tubular dilatation in C57BL/6J-APCMin/+ mice. MFC (Mouse forestomach carcinoma) tumors colonized the peritoneal cavity of mice. As the tumors grow, they may cause severe ascites accumulation and renal tubular damage in mice. It was found by experiments that intraperitoneal injection of 1.5×10⁷ MFC tumor cells can cause severe ascites accumulation on the 6th day, 24 hours after the injection of the tumor cells, continuous intraperitoneal injection of the NK3R inhibitor Pavinetant (50mg/kg/day) for 5 days can significantly inhibit the production of ascites and can alleviate renal tubular lesions, mainly including renal tubular dilation. Therefore, the NK3R inhibitor of the present invention can significantly restore tumor-induced renal tubular damage, and is conserved in various tumor models.

### Term Description:

Chronic kidney disease described in the present invention refers to: the phenotype of chronic kidney disease such as oliguria or ascites accumulation in the advanced stage of tumor patients.

Tumor-induced kidney disease or renal tubular damage in the present invention refers to: ascites accumulation, weight gain (mainly ascites increase), and renal tubular dilation in MFC tumor mice; reduced urine output and renal tubular dilation in Apc^{Min/+} mice; ascites accumulation or reduced urine output at patient's physiological level; renal tubular damage, including renal tubular dilation and changes in a series of molecular markers of damage at the tissue level.

### Brief Description of the Drawings

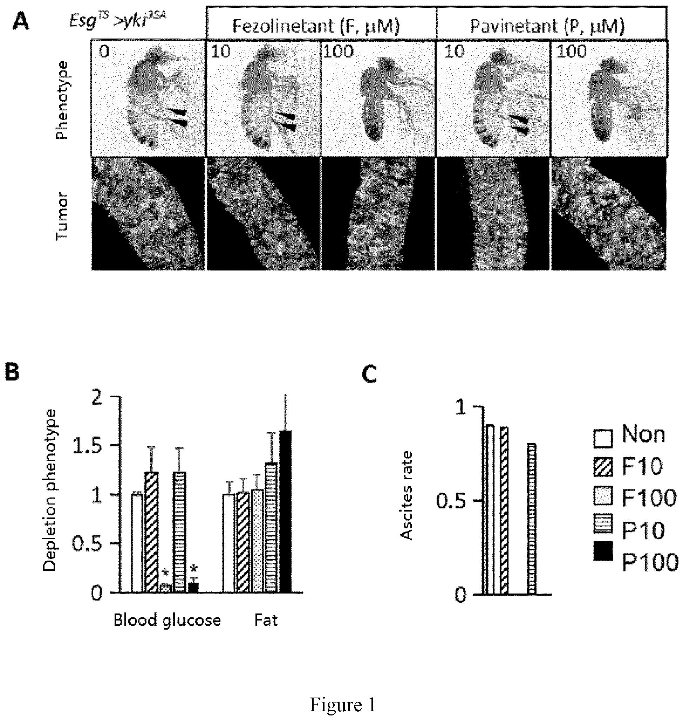

Figure 1. Effects of NK3R inhibitors on ascites in Drosophila caused by intestinal tumors;
   where panel A shows the ascites phenotype and tumor morphology of Drosophila; panel B shows the blood glucose and triglyceride levels of Drosophila; panel C shows the ascites ratio of Drosophila, Non is blank, F10 is Fezolinetant 10µM, F100 is Fezolinetant 100µM, P10 is Pavinetant 10µM, and P100 is Pavinetant 100µM.
Figure 2. NK3R expression in mouse renal tubules shown by immunohistochemistry;
   where the kidney of C57BL/6 mice were subjected to immunohistochemistry with the antibody ABclonalA7220 (left panel), and the antibody abcam ab124025 (right panel).
Figure 3. Effects of NK3R inhibitors on kidney damage caused by intestinal adenomas;
   Where panel E shows the body weight changes of C57BL/6 and C57BL/6J-APCMin/+ mice; panel F shows the urine output; panel G shows the HE staining of kidney longitudinal sections and F10 is Fezolinetant 10 mg/kg/day.
Figure 4. Effects of NK3R inhibitors on ascites and kidney damage caused by forestomach cancer cell tumors;
   where panel H shows the weight changes of C57BL/6 and MFC tumor mice; panel I shows the ascites volume of mice; panel J shows the expression level of the renal tubular damage marker gene NGAL; panel K shows the HE staining of kidney longitudinal sections, Pavi10 is Pavinetant 10 mg/kg/day, and Pavi50 is Pavinetant 50 mg/kg/day.

### Detailed Description of the Invention

The present invention is described in detail, but not limited, by the following description of preferred embodiments.

### Material Sources:

NK3R antibody 1: Abclonal company, Cat. No. A7220;
NK3R antibody 2: Abeam company, Cat. No. ab124025;
Fezolinetant: MedChemExpress company (MCE), Cat. No. HY-19632, 200 mg;
Pavinetant: MedChemExpress company (MCE), Cat. No. HY-14432, 1g, customized.
Experimental animals: Yki activated Drosophila (self-prepared in the laboratory, obtained from the cross of esg-GAL4, tub-GAL80TS, UAS-GFP and UAS-yki.3SA, literature published, pubmed No. 34407411)
C57BL/6J-APCMin/+ mice (Gempharmatech, Cat. No. T001457, genotype B6/JGpt-Apcem1Cin(Min)/Gpt)
Control C57BL/6J mice (Gempharmatech, Cat. No. N000013)
MFC tumor cells (ProCell, Cat. No. CL-0156, 1×10⁶ cells/T25 culture flask)
Other materials were purchased commercially.

### [Example 1] Effect of NK3R inhibitors on Drosophila ascites caused by intestinal tumors

Overexpression of the activated proto-oncogene yki in Drosophila intestinal stem cells can lead to malignant proliferation of intestinal stem cells, and then the formation of tumors. The formation of tumors seriously damages the secretory function of Drosophila Malpighian tubules, leading to the inability of circulating water in the Drosophila hemolymph to be drained out of the body, and the emergence of a severe ascites phenotype.

Methods: Activated yki tumors (genotypes esg-Gal4, UAS-GFP, tub-Gal80TS/+; UAS-yki3SA/+) were induced in the Drosophila intestine by initiating gene expression at 29°C. Drosophilas were fed with food supplemented with DMSO, Fezolinetant (10 µM, 100 µM), and Pavinetant (10 µM, 100 µM), respectively for 8 days, and compared with the control group (esg-Gal4, UAS-GFP, tub-Gal80TS/+), to observe phenotypes, detect intestinal tumor growth and systemic glycolipid changes, and count ascites ratios. See Figure 1.

Results: both the 100 µM NK3R inhibitors can significantly inhibit Drosophila ascites generation and restore blood glucose levels.

### [Example 2] High expression of NK3R in mouse renal tubules

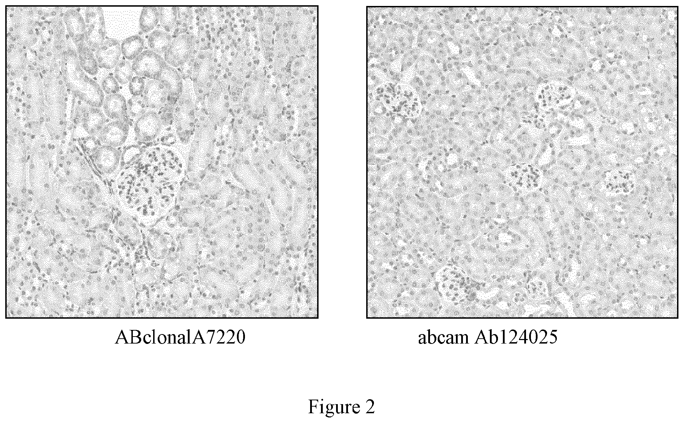

Methods: Kidney sections of C57BL/6J male mice aged 7-8 weeks old were taken and subjected to immunohistochemistry with two different NK3R antibodies (primary antibody dilution: ABclonalA7220, 1:200; abcamAb124025, 1:200), to observe the expression of NK3R in the kidneys. See Figure 2.

Results: NK3R was highly expressed in the renal tubules but not the glomeruli of mice.

### [Example 3] Effect of NK3R inhibitors on kidney damage caused by intestinal adenoma

C57BL/6J-APCMin/+ mice are a mature mammalian intestinal adenoma model and are commonly used to study host consumption caused by tumors. C57BL/6J-APCMin/+ mice develop severe renal tubular damage in the advanced stages of tumor development, and their urine output is significantly reduced compared to that of normal mice.

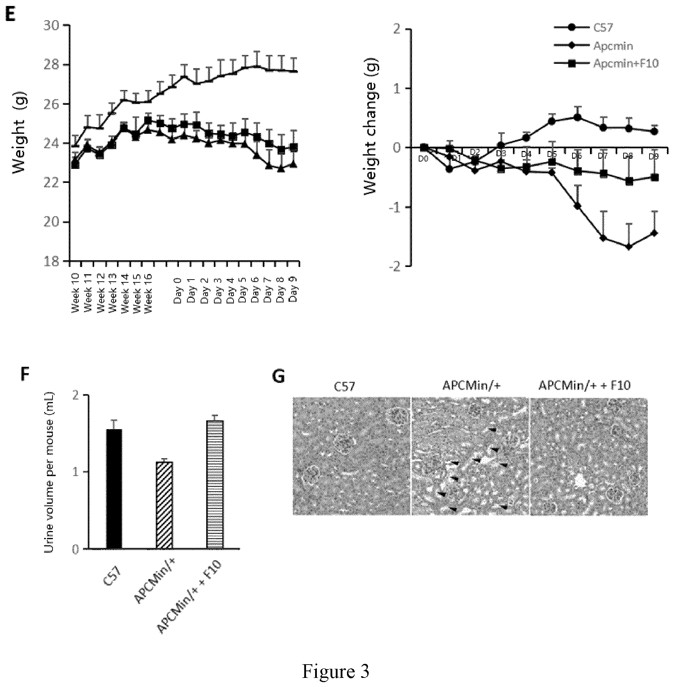

Methods: Male mice of C57BL/6J in the control group and C57BL/6J-APCMin/+ in the experimental group (7 mice in each group) were fed to 16 weeks of age under normal feeding conditions. When the mice in the experimental group began to lose weight, they were injected intraperitoneally with Fezolinetant (0, 10 mg/kg/day) for 9 consecutive days, and the body weight and urine output were measured, and the kidneys were stained to observe the tubular morphology. See Figure 3.

Results: Intraperitoneal injection of NK3R inhibitor Fezolinetant (10 mg/kg/day) significantly restored urine output of the C57BL/6J-APCMin/+ mice as well as their renal tubular damage.

### [Example 4] Effects of NK3R inhibitors on ascites and kidney damage caused by forestomach cancer cells

MFC tumor (Mouse forestomach carcinoma), a common tumor that may cause ascites, colonizes the peritoneal cavity of mice. With their growth, the tumors may cause severe ascites accumulation and renal tubular damage in mice.

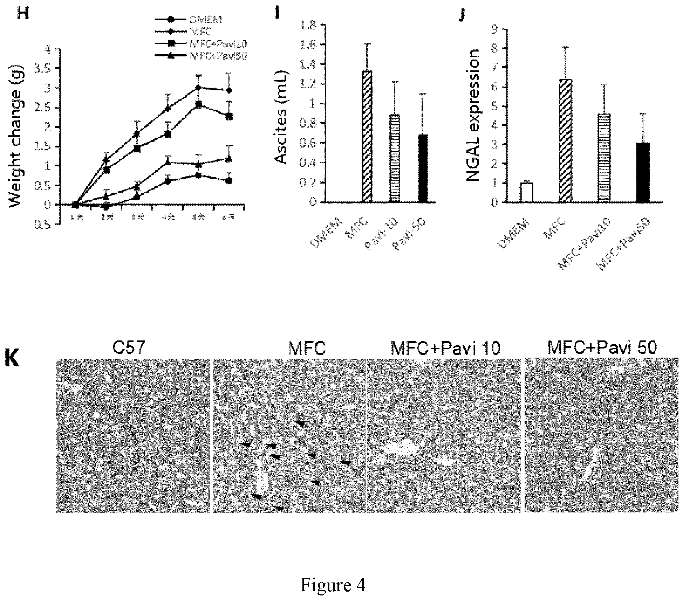

Methods: C57BL/6J male mice, 4-6 weeks old. C57BL/6J mice in experimental group (6 mice in each group) were intraperitoneally injected with 1.5×10⁷ MFC tumor cells, and 24 hours later, intraperitoneally injected with the inhibitor Pavinetant (0, 10, 50mg/kg/day), and after 5 days of continuous administration, they were compared with the control group C57BL/6 (6 mice in each group). The body weight, ascites, and urine output were detected, and the kidneys were stained to observe the tubular morphology. See Figure 4.

Results: The NK3R inhibitor Pavinetant (50mg/kg/day) can significantly inhibit the generation of ascites and alleviate renal tubular lesions.

### References:

[1] ZHU X, CALLAHAN M F, GRUBER K A, et al. Melanocortin-4 receptor antagonist TCMCB07 ameliorates cancer- and chronic kidney disease-associated cachexia[J]. J Clin Invest, 2020,130(9): 4921-4934.
[2] CUI Jiuwei, LI Wei, XU Hongxia, et al. Guidelines for the clinical diagnosis and treatment of tumor malignancy (2020) [J]. Chinese Journal of Clinical Oncology, 2021,48(08): 379-385.
[3] ROELAND E J, BOHLKE K, BARACOS V E, et al. Management of Cancer Cachexia: ASCO Guideline[J]. J Clin Oncol, 2020,38(21): 2438-2453.
[4] LAUNAY-VACHER V, JANUS N, DERAY G. Renal insufficiency and cancer treatments[J]. ESMO open, 2016,1(4): e91.
[5] MALYSZKO J, TESAROVA P, CAPASSO G, et al. The link between kidney disease and cancer: complications and treatment[J]. Lancet, 2020,396(10246): 277-287.
[6] ZHANG W, WANG Y, CHU Y. Tacr3/NK3R: Beyond Their Roles in Reproduction[J]. ACS Chemical Neuroscience, 2020,11(19): 2935-2943.
[7] GEORGE J T, KAKKAR R, MARSHALL J, et al. Neurokinin B Receptor Antagonism in Women With Polycystic Ovary Syndrome: A Randomized, Placebo-Controlled Trial[J]. The Journal of Clinical Endocrinology & Metabolism, 2016,101(11): 4313-4321.
[8] FRASER G L, LEDERMAN S, WALDBAUM A, et al. A phase 2b, randomized, placebo-controlled, double-blind, dose-ranging study of the neurokinin 3 receptor antagonist fezolinetant for vasomotor symptoms associated with menopause[J]. Menopause, 2020,27(4): 382-392.

## Claims

1. Use of a tachykinin receptor 3 inhibitor in the preparation of a drug for the treatment of chronic kidney disease, wherein the tachykinin receptor 3 inhibitor has a structure as shown in Formula (I) or Formula (II):

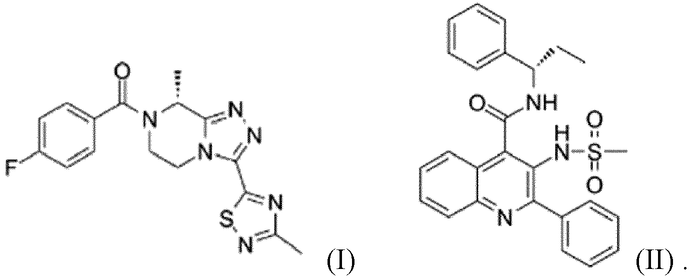

2. The use according to claim 1, wherein the chronic kidney disease is selected from glomerulonephritis, latent nephritis, pyelonephritis, allergic purpura nephritis, lupus erythematosus nephritis, gouty nephropathy, iga nephropathy, nephrotic syndrome, membranous nephropathy, nephrotic syndrome, diabetic nephropathy, hypertensive nephropathy, and polycystic kidney.

3. Use of a tachykinin receptor 3 inhibitor in the preparation of a drug for the treatment of a disease, wherein the disease is tumor-induced renal tubular damage or kidney disease, the tachykinin receptor 3 inhibitor has a structure as shown in Formula (I) or Formula (II):

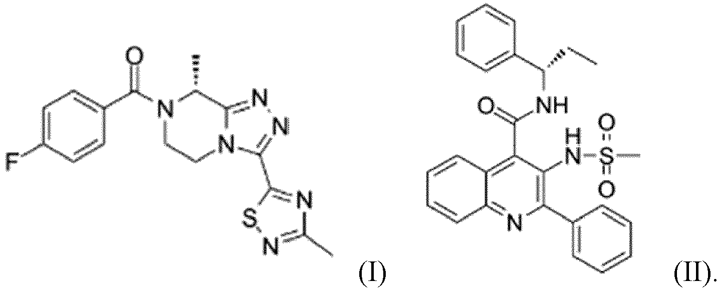

4. The use according to claim 3, wherein the disease is kidney damage in the advanced stage of tumor.
